# EUROPÄISCHE PATENTANMELDUNG

(11) **EP 1 695 634 A1**
(43) Veröffentlichungstag der Anmeldung: **30.08.2006**
(21) Anmeldenummer: 06002987.3
(22) Anmeldetag: 15.02.2006
(51) Int. Cl.: A23L 1/30, A61K 31/575, A23L 1/09

(54) **Zuckerhaltige Sterolfeststoffdispersionen**

(30) Priorität: 24.02.2005 DE 102005008445
(71) Anmelder: Cognis IP Management GmbH, 40589 Düsseldorf (DE)
(72) Erfinder: Schwarzer, Jörg, 40723 Hilden (DE); Salacz, Robert, 89250 Senden (DE); Horlacher, Peter, 89287 Bellenberg (DE); Kraus, Manuela, 89257 Illertissen (DE); Albiez, Wolfgang, 89257 Illertissen (DE)

(57) **Zusammenfassung**

Vorgeschlagen werden Feststoffdispersionen in Form fester Lösungen, enthaltend
a) mindestens 0, 1 Gew % Sterole und/oder Stanole und/oder deren Ester und
b) mindestens 50 Gew. % Zucker und/oder Zuckeralkohole
bezogen auf das Gesamtgewicht der Feststoffdispersion.

Diese Feststoffdispersionen ermöglichen eine einfache Weiterverarbeitung der hydrophoben Sterole und Sterolester durch ein verbessertes Dispergierverhalten in Lebensmitteln und zeichnen sich durch eine gute Lagerstabilität aus.

## Beschreibung

### Gebiet der Erfindung

Die Erfindung befindet sich auf dem Gebiet der Lebensmittel und betrifft Formulierungen zur Einarbeitung in Lebensmittel, kosmetische und pharmazeutische Zubereitungen, die Sterole, Stanole und/oder deren Ester enthalten, ein Verfahren zur Herstellung derselben sowie Zubereitungen, insbesondere Lebensmittel, die diese Formulierungen beinhalten.

### Stand der Technik

Phytosterole und deren durch Hydrierung gehärteten Derivate, Phytostanole, sind seit vielen Jahren bekannt für ihre cholesterinsenkenden Eigenschaften. Bereits 1991 konnte man ihre Wirkung über eine Hemmung der Absorption von Cholesterin im Darmbereich und über die inneren Blutgefässwände nachweisen.
Sterole und Stanole werden daher verbreitet in Lebensmitteln eingesetzt, denn sie minimieren durch ihre hypocholesterinämische Wirkung Folgekrankheiten wie Atherosklerose, Herzerkrankungen oder Bluthochdruck.
Da Phytosterole und -stanole in Wasser unlöslich und in Fetten und Ölen nur schlecht löslich sind, wirft die Einarbeitung der Cholesterinsenker in Lebensmittelzubereitungen, kosmetische oder pharmazeutische Produkte erhebliche Probleme auf. Aus dem ungünstigen Löslichkeitsverhalten der Substanzen resultiert neben der schlechten Dispergierbarkeit auch eine verminderte Bioverfügbarkeit und eine unbefriedigende Stabilität der Lebensmittelzubereitungen.

Zu den Bemühungen, dieses Problem zu lösen, gehörte die Formulierung von Estern der Sterole beispielsweise beansprucht in der Europäischen Patentanmeldung **EP 1275309 A1** oder Estern der Stanole wie im US-Patent **US 5,502,045** beschrieben, die durch ihre bessere Löslichkeit eine geringfügig verbesserte Verarbeitbarkeit aufwiesen jedoch gegenüber den freien Sterolen auch eine veränderte hypocholesterinämische Wirkung aufwiesen. Jedoch haben auch die veresterten Derivate noch ein unzureichendes Lösungsvermögen, um eine einfache Einarbeitung zu ermöglichen.

In zahlreichen Patentanmeldungen wird beschrieben, wie die Verfügbarkeit von Sterolen über die Reduktion der Partikelgrößen vornehmlich durch Mikronisation verbessert werden kann. So beschreibt die deutsche Offenlegungsschrift **DE 102 53 111 A1** pulverförmige Phytosterol-Formulierungen mit einer mittleren Teilchengröße von 0,01 bis 100 µm, die sich gut in Wasser redispergieren lassen. Einen Prozess zur Herstellung einer Steroldispersion, in der die Partikelgrößenverteilung der Sterole bei 0,1 bis 30 µm liegt entnimmt man der Internationalen Anmeldung **WO 03/105611 A2.**

Häufig ist jedoch die Mikronisation der Sterolpartikel alleine nicht ausreichend, um eine gute Einarbeitbarkeit zu erreichen. Zwar lässt sich durch die Erhöhung der Oberfläche die Bioverfügbarkeit der fein dispergierten Teilchen verbessern, jedoch sind gerade die mikronisierten Partikel schlecht benetzbar, aggregieren leicht und schwimmen auf wässrigen Oberflächen meistens auf. Häufig lässt sich das gemahlene Sterol nur mit speziellen Methoden in einem Getränk dispergieren, wozu intensives Mischen notwendig ist. Diese Geräte stehen jedoch im Normalfall dem Endanwender der Lebensmittelhersteller nicht zur Verfügung.

Daher kombinieren viele Hersteller die Mikronisation der Sterole mit der zusätzlichen Anwendung von Emulgatoren. Ein Beispiel dafür sind die in dem Europäischen Patent **EP 0897671 B1** beanspruchten Zubereitungen mit Sterolen und Sterolestern mit einer Partikelgröße von maximal 15 µm in einem Gemisch mit Emulgatoren, wobei das Gewichtsverhältnis Emulgator zu Sterol in der wässrigen Phase weniger als 1:2 ist. Eine in der Europäischen Anmeldung **EP 1142494 A1** offenbarte Sterol-Emulgator-Dispersion hat eine Teilchengrößenverteilung von 1 bis 40 µm.
Häufig verwendete Emulgatoren sind Monoglyceride und Polysorbate **[US 6,623,780 B1, US 6,376,482 B2, WO 02/28204 A1].** Auch wenn diese sich durch eine gute Verträglichkeit auszeichnen und als Lebensmittelemulgatoren über einen langen Zeitraum bereits bekannt sind, versucht man die Menge der Emulgatoren zu verringern oder sie gar ganz zu vermeiden, da Emulgatoren auch die Bioverfügbarkeit weiterer in den Lebensmitteln vorhandener Substanzen beeinflussen oder die Stabilität der Formulierungen negativ verändern können.
Die Vermeidung von Emulgatoren war auch Ziel der in dem Europäischen Patent **EP 1059851 B1** offenbarten Sterolformulierungen, die Verdicker zur besseren Dispergierbarkeit enthalten.

Zahlreiche weitere Methoden zur Verbesserung der Löslichkeit und Dispergierbarkeit wie die Formulierung als Emulsionen, Microemulsionen, Dispersionen, Suspensionen oder die Komplexierung mit Cyclodextrinen oder Gallensalzen werden in der Internationalen Patentanmeldung **WO 99/63841 A1** vorgestellt, darunter auch die Formulierung in Form von Feststoffdispersionen. Als Träger werden PEG, PVP, Copolymere, Celluloseether und ester vorgeschlagen.
Auch in der Europäischen Patentanmeldung **EP 1074185 A1** werden Sterolhaltige Formulierungen mit einer Matrix beschrieben, die sich durch vorteilhafte organoleptische Eigenschaften der Zubereitungen auszeichnen und für Süsswaren eingesetzt werden. Diese Formulierungen werden jedoch unter Zugabe von Wasser oder Zugabe von Wasser und Emulgatoren hergestellt und haben daher eine verringerte Haltbarkeit.

Aufgabe der vorliegenden Erfindung war es, eine Formulierung zur Verfügung zu stellen, die eine einfache und gute Dispergierung und Einarbeitung von Sterolen, Stanolen und deren Estern in Lebensmitteln ermöglicht, wobei die Anwendung von Emulgatoren reduziert oder ganz vermieden werden soll. Diese Sterolformulierung sollte einfach herzustellen sein und sich durch eine gute Lagerstabilität auszeichnen.

### Beschreibung der Erfindung

Gegenstand der Erfindung sind Feststoffdispersionen, in Form einer festen Lösung enthaltend
a) mindestens 0,1 Gew % Sterole und/oder Stanole und/oder deren Ester
b) mindestens 50 Gew. % Zucker und/oder Zuckeralkohole
bezogen auf das Gesamtgewicht der Feststoffdispersion.

Die erfindungsgemäßen Feststoffdispersionen zeichnen sich durch gute Solubilisierungseigenschaften, verminderte Aggregations- und Agglomerationseigenschaften und eine verbesserte Benetzbarkeit aus und ermöglichen so eine einfache Dispergierung von Sterolen, Stanolen und deren Estern in wasser- und fetthaltigen Zubereitungen. Sie lassen sich hervorragend handhaben, da sie in den unterschiedlichsten Teilchengrößen verfügbar sind und ohne aufwendige Ausrüstung weiterverarbeitetet werden können und zeigen außerdem eine gute Lagerstabilität.

Die so zusammengesetzten Feststoffdispersionen in Form fester Lösungen können auf einfache Weise in Lebensmittel, insbesondere in Milch, Milchgetränke, Molke-, Joghurtgetränke, Margarine, Fruchtsäfte, Fruchtsaftgemische, Fruchtsaftgetränke, Gemüsesäfte, Kohlensäurehaltige und Kohlensäurefreie Getränke, Sojamilchgetränke oder proteinreiche flüssige Nahrungsersatzgetränke, sowie fermentierte Milchzubereitungen, Joghurt, Trinkjoghurt, oder Käsezubereitungen, aber auch in kosmetische oder pharmazeutische Zubereitungen eingearbeitet werden.
Ein weiterer Gegenstand der Erfindung sind daher Zubereitungen, die die Feststoffdispersionen der genannten Zusammensetzung enthalten. Sie werden bevorzugt in Getränken und Milchprodukten eingesetzt, die dann 0,1 bis 50 Gew. %, bevorzugt 1 bis 20 Gew. % der Feststoffdispersionen bezogen auf das Gesamtgewicht der Lebensmittel, enthalten.

### Sterole, Stanole und deren Ester

In der vorliegenden Erfindung werden aus Pflanzen und pflanzlichen Rohstoffen gewonnene Sterole, sogenannte Phytosterole und Phytostanole sowie Ester der Phytosterole und Ester der Phytostanole eingesetzt. Bekannte Beispiele sind Ergosterol, Brassicasterol, Campesterol, Desmosterol, Clionasterol, Stigmasterol, Poriferasterol, Chalinosterol, Sitosterol und deren Mischungen, darunter werden bevorzugt β- Sitosterol und Campesterol verwendet. Ebenso fallen die hydrierten gesättigten Formen der Sterole, die sogenannten Stanole unter die eingesetzten Verbindungen, auch hier werden β-Sitostanol und Campestanol bevorzugt.
Weiterhin werden Ester der Phytosterole und Ester der Phytostanole in den erfindungsgemäßen Formulierungen eingesetzt. Auch diese Derivate der Sterole und Stanole sind noch nicht ausreichend hydrophil und benetzbar, um in Lebensmittel oder andere Zubereitungen einfach einarbeitbar zu sein. Es werden bevorzugt Veresterungsprodukte mit gesättigten und/oder ungesättigten Fettsäuren mit 6 bis 22 und vorzugsweise 12 bis 18 Kohlenstoffatomen verarbeitet. Die Erfindung ist jedoch nicht begrenzt auf diese Art der Ester.
Besonders bevorzugt werden jedoch unveresterte Sterole eingesetzt, die bedingt durch die Herstellung geringe Mengen an Stanolen enthalten.

### Zucker und Zuckeralkohole

Die als Zucker eingesetzten Verbindungen beinhalten alle lebensmittelgeeigneten Zucker, ausgewählt aus der Gruppe, die gebildet wird von Glucose, Saccharose, Fructose, Trehalose, Maltose, Maltodextrin, Cyclodextrin, Invertzucker, Palatinose und Lactose. Bevorzugt werden Glucose oder Saccharose zugesetzt.
Auch die eingesetzten Zuckeralkohole müssen toxikologisch unbedenklich und nahrungsmittelgeeignet sein. Es werden Zuckeralkohole ausgewählt aus der Gruppe, die gebildet wird von Sorbitol, D-Mannitol, Maltitol, Lactitol, Isomalt und Xylitol, darunter besonders bevorzugt Sorbitol verwendet. Die geeigneten Mengen der Zucker und/oder Zuckeralkohole in den erfindungsgemäßen Feststoffdispersionen belaufen sich auf mindestens 50 Gew.% bezogen auf das Gesamtgewicht der Feststoffdispersion. Bevorzugt werden mindestens 80 Gew. % besonders bevorzugt mindestens 90 Gew. % eingesetzt.

### Feststoffdispersionen in Form fester Lösungen und deren Herstellung

Bei den erfindungsgemäßen Feststoffdispersionen in Form fester Lösungen handelt es sich um Lösungen von festen Stoffen in festen Stoffen, durch die eine sehr feine Verteilung von Sterolen, Stanolen oder deren Estern im Zucker und/oder Zuckeralkohol gewährleistet ist, da die Sterole in einer Matrix eingebettet vorliegen. Die Verteilung der dispersen Phase kann hierbei grobdispers, kolloiddispers oder molekulardispers ausfallen. Feststoffdispersionen in Form fester Lösungen sind wasserfrei, sie enthalten weniger als 0,5 Gew. % vorzugsweise weniger als 0,3 Gew. % und besonders bevorzugt weniger als 0,1 Gew. % Wasser.

Diese Formulierungen unterscheiden sich in ihrem physikalisch-chemischen Verhalten deutlich von einfachen Pulvergemischen, die durch Mischen feinteiliger Pulver entstehen, wie sie beispielsweise in der Internationalen Patentanmeldung **WO 98/13023** im Beispiel 3 beschrieben sind, die noch zwei physikalisch getrennte Komponenten darstellen. Bei den erfindungsgemäßen Feststoffdispersionen liegen die Sterole, Stanole und deren Ester auch bei vergleichbarer kleiner Partikelgrößenverteilung in eine Matrix eingeschlossen vor.

Auch in der Europäischen Patentanmeldung **EP1074185 A1** werden Sterolhaltige Formulierungen mit einer Matrix beschrieben, die sich durch vorteilhafte organoleptische Eigenschaften der Zubereitungen auszeichnen und für Süsswaren eingesetzt werden. Diese Formulierungen werden jedoch unter Zugabe von Wasser oder Zugabe von Wasser und Emulgatoren hergestellt und stellen daher keine typische Feststoffdispersion dar.

Im Gegensatz zu den physikalischen Pulvermischungen und wasserhaltigen Matrixsystemen zeichnen sich die erfindungsgemäßen Feststoffdispersionen durch eine gute Lagerstabilität, sehr gute Solubilisierungseigenschaften, verminderte Aggregations- und Agglomerationseigenschaften und eine verbesserte Benetzbarkeit aus.

Grundsätzlich lassen sich Feststoffdispersionen über die Schmelz- oder Lösemethode herstellen. Aufgrund der unterschiedlichen Löslichkeitseigenschaften von Zuckern und Sterolen kann bei den vorliegenden Feststoffdispersionen nur die Schmelzmethode eingesetzt werden, bei der die Sterole, Stanole und/oder deren Ester mit den Zuckern und/oder Zuckeralkoholen zusammen aufgeschmolzen werden und beim Abkühlen erstarren. Hierbei kann das Abkühlen der aufgeschmolzenen Masse unter Rühren oder beim Kneten erfolgen.
Möglich ist auch eine gleichzeitige Extrusion. Dabei tritt die Masse aus dem Extruder bedingt durch Scherkräfte warm heraus und wird nachfolgend abgekühlt. Dieses kann beispielsweise auf Kühlbändern erfolgen.

Die erfindungsgemäßen Feststoffdispersionen in Form fester Lösungen sind erhältlich dadurch, dass man
a) Sterole und/oder Stanole und/oder deren Ester bei Temperaturen von 60 bis 190 °C zusammen mit einem Zucker und/oder Zuckeralkohol aufschmilzt und
b) die Schmelze langsam auf Raumtemperatur abkühlen lässt.
Danach kann die erstarrte Schmelze gegebenenfalls auf die gewünschte Partikelgröße zerkleinert werden.
Das Aufschmelzen der Komponenten findet ohne Zugabe von Wasser statt. Die entstehende Schmelze wird bis zum vollständigen Aufschmelzen der Komponenten auf der Temperatur von 60 bis 190°C, vorzugsweise 100 bis 190 C°, besonders bevorzugt 120-180°C gehalten.

Vorzugsweise erhält man die erfindungsgemäßen Feststoffdispersionen in Form fester Lösungen dadurch, dass man
a) mindestens 0,1 Gew. % (bezogen auf das Gesamtgewicht der Endformulierung) Sterole und/oder Stanole und/oder deren Ester bei Temperaturen von 60 bis 190°C zusammen mit mindestens 50 Gew. % (bezogen auf das Gesamtgewicht der Endformulierung) Zucker und/oder Zuckeralkohol aufschmilzt,
b) die Schmelze unter Rühren langsam auf Raumtemperatur abkühlen lässt und
c) gegebenenfalls durch Mahlen auf eine mittlere Teilchengröße von 125 µm und größer zerkleinert.

### Teilchengrößenverteilung

Die Teilchengröße der unbehandelten Sterine vor der Herstellung der Feststoffdispersionen ist für die Endformulierung nicht wesentlich, was die Verarbeitung sehr vereinfacht. Hier können Schuppen, Prills, Pastillen, sogar auch Blockware verwendet werden, da das Produkt mit Zucker aufgeschmolzen wird.
Nach Erstarren der Feststoffdispersionen können die Formulierungen zu der gewünschten Teilchengröße mit üblichen Zerkleinerungsgeräten vermahlen werden. Dabei muss keine Mikronisation der Feststoffdispersionen erfolgen, da sogar bei Teilchengrößenverteilungen von 125 bis 500 µm die eingebetteten Sterolpartikel nach Auflösung des Zuckers oder Zuckeralkohols in mikronisierter Form vorliegen. Auch hier eignen sich Formulierungen in Form von Schuppen, Prills, Pastillen oder Blockware zur vereinfachten Weiterverarbeitung.
Es wurden für die Feststoffdispersionen Teilchengrößen von 125 µm, 250 µm und 500 µm durch Vermahlen eingestellt. Je größer die Teilchengröße der Feststoffdispersionen ausfiel, desto besser ließ sich das Pulver handhaben. Bevorzugt werden Pulver mit einer mittleren Teilchengröße von 125 µm bis 4000µm - gemessen mit Siebanalyse gemäss Europäischem Arzneibuch (EuAB): Hauptfraktion der Siebung mit Siebgrößen der lichten Maschenweite von 90, 125, 250, 500, 1000 und 4000 µm - besonders bevorzugt haben die Pulver eine mittleren Teilchengröße von 250 µm bis 1000 µm und speziell werden Pulver mit Teilchengrößen von 250 bis 500 µm bevorzugt.

### Rezepturen für Zuckerhaltige Feststoffdispersionen

Erfindungsgemäße Zubereitungen sind:
**A)** Feststoffdispersionen in Form fester Lösungen, enthaltend
   a) mindestens 0,1 Gew % Sterole und/oder Stanole und/oder deren Ester
   b) mindestens 50 Gew. % Zucker und/oder Zuckeralkohole
   bezogen auf das Gesamtgewicht der Feststoffdispersion.
**B)** Feststoffdispersionen in Form fester Lösungen, enthaltend
   a) mindestens 1 Gew % Sterole und/oder Stanole und/oder deren Ester
   b) mindestens 80 Gew. % Zucker und/oder Zuckeralkohole
   bezogen auf das Gesamtgewicht der Feststoffdispersion.

Aufgrund der verbesserten Lagerstabilität, Dispergierbarkeit und Verarbeitbarkeit in Lebensmitteln werden die folgenden Feststoffdispersionsrezepturen bevorzugt:
**C)** Feststoffdispersionen in Form fester Lösungen, enthaltend
   a) mindestens 3 Gew % Sterole und/oder Stanole und/oder deren Ester
   b) mindestens 90 Gew. % Zucker und/oder Zuckeralkohole
   bezogen auf das Gesamtgewicht der Feststoffdispersion.
**D)** Feststoffdispersionen in Form fester Lösungen, enthaltend
   a) mindestens 1 Gew % Sterole und/oder Stanole und/oder deren Ester
      b) mindestens 80 Gew. % Zucker und/oder Zuckeralkohole und
      c) weniger als 0,5 Gew. % Wasser
   bezogen auf das Gesamtgewicht der Feststoffdispersion.
**E)** Feststoffdispersionen in Form fester Lösungen, enthaltend
   a) mindestens 0,1 Gew % Sterole und/oder Stanole und/oder deren Ester
      b) mindestens 50 Gew. % Zuckeralkohole
   bezogen auf das Gesamtgewicht der Feststoffdispersion.

Besonders bevorzugt werden die Feststoffdispersionen in der folgenden Zusammensetzung:
**F)** Feststoffdispersionen in Form fester Lösungen, enthaltend
   a) mindestens 1 Gew % Sterole und/oder Stanole und/oder deren Ester und
   b) mindestens 80 Gew. % Zuckeralkohole
   bezogen auf das Gesamtgewicht der Feststoffdispersion.
G) Feststoffdispersionen in Form fester Lösungen, enthaltend
   a) mindestens 1 Gew % Sterole und/oder Stanole und/oder deren Ester
      b) mindestens 80 Gew. % Zucker und/oder Zuckeralkohole und
      c) weniger als 0,3 Gew. % Wasser
   bezogen auf das Gesamtgewicht der Feststoffdispersion.
**H)** Feststoffdispersionen in Form fester Lösungen, enthaltend
   a) mindestens 3 Gew % Sterole und/oder Stanole und/oder deren Ester
      b) mindestens 90 Gew. % Zucker und/oder Zuckeralkohole und
      c) weniger als 0,5 Gew. % Wasser
   bezogen auf das Gesamtgewicht der Feststoffdispersion.

Speziell bevorzugt werden die Emulsionen in der folgenden Zusammensetzung:
**I)** Feststoffdispersionen in Form fester Lösungen, enthaltend
   a) mindestens 1 Gew % Sterole und/oder Stanole und/oder deren Ester und
   b) mindestens 50 Gew. % Sorbitol
   bezogen auf das Gesamtgewicht der Feststoffdispersion.
**J)** Feststoffdispersionen in Form fester Lösungen, enthaltend
   a) mindestens 1 Gew % Sterole und/oder Stanole und/oder deren Ester
   b) mindestens 80 Gew. % Zucker und/oder Zuckeralkohole und
   c) weniger als 0,1 Gew. % Wasser
   bezogen auf das Gesamtgewicht der Feststoffdispersion.
**K)** Feststoffdispersionen in Form fester Lösungen, enthaltend
   a) mindestens 3 Gew % Sterole und/oder Stanole und/oder deren Ester
      b) mindestens 90 Gew. % Zucker und/oder Zuckeralkohole und
      c) weniger als 0,3 Gew. % Wasser

Die beste Lagerstabilität wiesen Formulierungen der folgenden Zusammensetzung auf:
**L)** Feststoffdispersionen in Form fester Lösungen, enthaltend
   a) mindestens 3 Gew % Sterole und/oder Stanole und/oder deren Ester
      d) mindestens 90 Gew. % Zucker und/oder Zuckeralkohole und
      e) weniger als 0,1 Gew. % Wasser
bezogen auf das Gesamtgewicht der Feststoffdispersion.

### Beispiele

### Beispiel 1: Versuche mit Zucker - Saccharose

25 g Generol® 122 N in Form von Prills mit einer durchschnittlichen Partikelgröße von 500 - 800µm (Cognis Deutschland) und 225 g handelsüblicher Rohrzucker - Saccharose - (Südzucker RFF) wurden gemeinsam geschmolzen und gerührt. Anschließend wurde die Schmelze unter manuellem Kneten abgekühlt. Die erstarrte Masse wurde dann mit einer herkömmlichen Haushaltsmühle (Moulinette®) vermahlen zu einer durchschnittlichen Teilchengröße von A: 125 µm und B: 250 µm und C: 500 µm (Siebanalyse auf einer Siebanlage der Fa. Retsch, Hauptmenge Siebanalyse gemäß EuAB).

### Dispergiertest:

Das so erhaltene Pulver mit einem Gehalt von 10 Gew.% Sterol wurde in Orangensaft, Milch und Wasser im Vergleich zu gemahlenen Sterolen (Generol® 122NG mittlere Teilchengröße 20-40µm, bestimmt durch Laserbeugungsverfahren) dispergiert. Hierzu wurden 250 ml der zu untersuchenden Flüssigkeit in ein Becherglas gegeben und gerührt (ca. 100 rpm). Zu der gerührten Flüssigkeit wurde soviel Pulver (A, B, C und Vergleich) zugegeben, dass die Dispersion einen Gehalt von 0,5 % Sterol aufwies und das Dispergierverhalten bewertet.

| | Sterol -Zuckerdispersion | unbehandelt (Vergleich) |
|---|---|---|
| O-Saft | gut (A, B, C) | schlecht |
| Wasser kalt | gut (A, B, C) | schlecht |
| Wasser warm | gut (A, B, C) | schlecht |
| Milch | gut (A, B, C) | schlecht |

Das unbehandelte Sterol ist von Natur aus hydrophob, es wird nicht benetzt und bleibt an der Oberfläche liegen. Die Sterol-Zucker-Dispersionen A, B und C wurden von der Flüssigkeit gut benetzt, der Zucker löste sich bei allen drei Formulierungen relativ schnell auf und gab das feinteilige Sterol dispergiert frei. Je größer die Teilchengröße war, desto einfacher ließ sich das Pulver handhaben.

### Teilchengrößenanalyse:

Die Teilchengrößenbestimmung der groberen Teilchen wurde durch Siebgrößenanalyse (gemäß Europäischem Arzneibuch) durchgeführt. Folgende Siebgrößen wurden verwendet:
Lichte Maschenweite in µm: 90, 125, 250, 500, 1000, 4000.
Bei mittleren Teilchengrößen unterhalb von 30 µm ist eine einfache trockene Siebung zur Größenbestimmung nicht mehr möglich. In diesem Fall wurde die Partikelgrößenbestimmung mit einem Laserbeugungsverfahren durchgeführt: Die Teilchengrößenverteilung wurde mit einem Gerät der Fa. Beckman Coulter, Typ LS 230 gemäß Bedienungsanleitung (1994) bestimmt. Als Messmedium wurde Wasser verwendet.

Die Teilchengrößenmessungen der Endformulierungen erfolgten unmittelbar nach der Herstellung der Dispersionen.
Im Versuch wurden Prills als Ausgangsstoff für die reinen Sterole verwendet, mit der Teilchengröße 500 -800µm (Hauptmenge der Verteilung: Siebanalyse EuAB)
Teilchengröße der gemahlenen Sterol-Zucker-Feststoffdispersionen:
Hauptmenge A: 125 µm und B: 250 µm und C: 500 µm Siebanalyse)
Dispergiert in Wasser lag das Maximum der Teilchengrößenverteilung bei 20-50µm (ermittelt durch Laserbeugung) Der Zucker löste sich somit im Wasser auf und die Teilchengrößenverteilung der reinen Sterolpartikel wurden vermessen.

### Beispiel 2: Versuche mit Zuckeralkohole - Sorbit

Mit Zuckeralkoholen, insbesondere Sorbit, erhalten wir zunächst nach dem gemeinsamen Schmelzen eine pastenähnliche Verbindung, die im Kalten erstarrt.
Dieser Feststoff kann jedoch wieder durch Erwärmen verflüssigt werden und z. B. zu kaltem Wasser zugegeben werden. Das Sorbitol löst sich auf und das Sterol ist fein dispergiert.

### Beispiel 2a:

25 g ungemahlenes Sterol (Generol® 122 N (Cognis Deutschland) ) werden gemeinsam mit 225 g Sorbitol (70%) geschmolzen. Anschließend wird die Masse unter Rühren oder Kneten (Bedingungen gemäß Beispiel 1) abgekühlt bis sie sich verfestigte.

Dispergiertest gemäss Beispiel 1:
- kaltes Wasser gut
- warmes Wasser gut
- Milch gut
- O-saft gut

### Beispiel 2b:

Der entsprechend Beispiel 2a durchgeführte Versuch wurde wiederholt mit auf 75°C erwärmter Feststoffdispersion. Diese Dispersion weist ein pastenähnliches Fließverhalten auf. Ergebnis:
Dispergiertest
   - kaltes Wasser gut
   - warmes Wasser gut
   - Milch gut
   - O-saft gut

## Patentansprüche

1. Feststoffdispersionen in Form einer festen Lösung enthaltend
a) mindestens 0,1 Gew % Sterole und/oder Stanole und/oder deren Ester
b) mindestens 50 Gew. % Zucker und/oder Zuckeralkohole
bezogen auf das Gesamtgewicht der Feststoffdispersion.

2. Feststoffdispersionen gemäss Anspruch 1, **dadurch gekennzeichnet, dass** sie als Pulver mit einer mittleren Teilchengröße von 125 µm bis 4000 µm - gemessen durch Siebanalyse - vorliegen.

3. Feststoffdispersionen gemäß den Ansprüchen 1 und/oder 2, **dadurch gekennzeichnet dass** sie in Form von Schuppen, Prills, Pastillen oder Blockware vorliegt.

4. Feststoffdispersionen, gemäß den Ansprüchen 1 bis 3, **dadurch gekennzeichnet, dass** sie als Komponente b) Zucker ausgewählt aus der Gruppe, die gebildet wird von Glucose, Saccharose, Fructose, Trehalose, Maltose, Maltodextrin, Cyclodextrin, Invertzucker, Palatinose und Lactose enthalten.

5. Feststoffdispersionen, gemäß den Ansprüchen 1 bis 3, **dadurch gekennzeichnet, dass** sie als Komponente b) Zuckeralkohole ausgewählt aus der Gruppe, die gebildet wird von Sorbitol, D-Mannitol, Maltitol, Lactitol, Isomalt und Xylitol enthalten.

6. Feststoffdispersionen, gemäß den Ansprüchen 1 bis 5, **dadurch gekennzeichnet, dass** sie als Komponente b) Saccharose und bevorzugt Sorbit enthalten.

7. Zubereitungen enthaltend Feststoffdispersionen gemäß der Ansprüche 1 bis 6.

8. Getränke und Milchprodukte, enthaltend 0,1 bis 50 Gew.% der Feststoffdispersionen gemäß der Ansprüche 1 bis 6 bezogen auf das Gesamtgewicht der Sterol-haltigen Lebensmittel.

9. Verfahren zur Herstellung von zuckerhaltigen Sterolfeststoffdispersioen , bei dem man
a) Sterole und/oder Stanole und/oder deren Ester bei Temperaturen von 60 bis 190 °C zusammen mit einem Zucker oder Zuckeralkohol aufschmilzt,
b) die Schmelze unter Rühren langsam auf Raumtemperatur abkühlen lässt und
c) gegebenenfalls durch Mahlen auf eine mittlere Teilchengröße von 125 µm bis 4000 µm zerkleinert.
